# EUROPEAN PATENT SPECIFICATION

(11) **EP 0 381 181 B1**
(45) Date of publication and mention of the grant of the patent: **09.08.1995**
(21) Application number: 90101888.7
(22) Date of filing: 31.01.1990
(51) Int. Cl.: A61K 9/52, A61K 9/36

(54) **A system for the controlled release of active agents and a process for its preparation**
System zur gesteuerten Freisetzung von Wirkstoffen sowie Verfahren zu dessen Herstellung
Système de libération contrôlée de substances actives et son procédé de préparation

(30) Priority: 31.01.1989 DD 325354; 03.10.1989 DE 3932987
(43) Date of publication of application: 08.08.1990
(73) Proprietor: IPA INTERNATIONALE PHARMA AGENTUR GMBH, 89231 Neu-Ulm (DE)
(72) Inventor: Wenzel, Udo, Prof. Dr. sc. nat., DDR-4050 Halle/Saale (DE); Metzner, Jürgen, Doz. Dr.sc.med.Dr.rer.nat., DDR-4020 Halle/Saale (DD); Rosin, Thomas, Dr., DDR-4090 Halle/Neustadt (DD); Jaeger, Halvor, Dr.med., D-7910 Neu-Ulm/Gerlenhofen (DE); Salama, Zoser B., Dr.rer.nat., D-7913 Senden/Wullenstetten (DE)
(74) Representative: VOSSIUS & PARTNER

(56) References cited:
- EP-A- 0 238 189
- EP-A- 0 279 976
- GB-A- 2 150 434

## Description

The invention relates to a system for the controlled release of active agents and a process for its preparation. The system is suitable for various kinds of active agents and will be explained in the following using a preferred embodiment, i.e. the release of a pharmaceutical agent, as an example.

The system is preferably used for orally administerable pharmaceutical preparations, especially such that are soluble, operating with essentially even, controlled pharmaceutical agent release which is effected by convective processes.

As regards its therapeutic effectivity, the ideal oral delayed release pharmaceutical preparation must be similar in effectivity to an intravenous drip. In other words, due to the even and complete release of the active agent from the pharmaceutical preparation for a physiologically and therapeutically practical period of time a constant, therapeutic blood level is obtained. This level guarantees the long-term effect of the drug whilst reducing the adverse effects and improving the local tolerance in the gastrointestinal tract at the same time.
Pharmaceutical preparations in which the active agent release is controlled, i.e. it is independent of marginal conditions, are considered especially advantageous. This is because in such pharmaceutical preparations the constant active agent release is ensured with a high measure of certainty and the in vitro and in vivo release rates are compatible.

It is common knowledge that the release of active agents can be controlled by embedding processes and/or matrix, covering or coating processes as well as by osmotically driven systems.

Matrix processes as described, for example, in DD-A-232 821, have the drawback that they usually cannot be used to obtain pharmaceutical agent release according to zero-order kinetics. The literature on release systems on a matrix basis is extensive (e.g. S.D. Bruck, Controlled Drug Delivery, Vol. I and II, CRC-Press (1983)).

As regards covered pharmaceutical preparations, it is necessary to find a suitable shell that will control the diffusion of the dissolved pharmaceutical agent according to the physico-chemical properties of the active agent; today it is almost always polyacrylic acid esters-co-methacrylic acid esters that are used for this purpose.

Whilst it is easier and more accurate to realise the pharmacokinetically favourable release of the zero-order using covered pharmaceutical preparations, they have the enormous drawback that any damage to the shell results in the entire dosis incorporated in the pharmaceutical preparation being released within a short period of time (dose dumping). This, in turn, causes significant adverse effects in the patient and jeopardises therapy safety.

In an attempt to increase the safety level of the conventional single unit delayed release preparation, the total dosis was split up into several smaller partial delayed release doses (multiple unit dosage forms) under acceptance of an extreme increase in technological expenditure (S.D. Bruck, Controlled Drug Delivery, Vol. I and II, CRC-Press (1983); R. Baker, Controlled Release of Bioactive Material, Acad. Press (1980); Y.W. Chien, Novel Drug Delivery Systems, Marcel Dekker (1982); J.R. Robinson, Sustained and Controlled Release Drug Delivery Systems, Marcel Dekker (1978)).

The osmotically driven systems described, for instance, by Theeuws in J. Pharm. Sc. Vol. 64, 12, pages 1987 to 1991 or in DE-A-26 40 193 and EP-A-0 169 105 exhibit an almost ideal release rate which is independent of marginal conditions and permits the maintenance of the desired serum level for a therapeutically and physiologically practical period of time.

In principle, osmotic systems have one or more release openings whose preparation (e.g. by means of laser drills) usually entails high technological costs. An exact description of the preparation of the release openings and their minimum and maximum dimensions can be found in US-A-3,845,770 and in 3,916,899 plus the figures contained therein.

The serious side effects which occurred in medication with osmotic systems and led to the withdrawal of these osmotically releasing pharmaceutical preparations for Indomethacin (see Martin, A.N., Physikalische Pharmazie, Ed. H. Stricker, 3rd Edition, page 576, Stuttgart 1987) were said to be caused by the concentrated release of the pharmaceutical agent from the release opening or openings (the so-called flame-cutter effect).

Thus there is a need for the provision of a system for the controlled release of high solubility active agents and a process for its preparation, preferably for high solubility pharmaceutical agents.

The object underlying the invention is to provide a system for the controlled release of active agents in which, in the case of a pharmaceutical agent, the release of the active agent occurs throughout a physiologically and. therapeutically practical period of time under zero-order kinetics and, as far as possible, is independent of the marginal conditions. Furthermore, by means of appropriate measures the lag time commonly characteristic of osmotically releasing systems is to be selectively adjustable, and the release pattern is to remain unchanged, at least essentially, even if the controlling membrane that controls the release rate is damaged.
Release is preferably to occur over the entire surface of the pharmaceutical preparation in order to prevent damages caused by the emergence of highly concentrated pharmaceutical preparation-adjuvant solutions at one site. The delayed release of the active agent is to occur by means of suitable adjuvants that are industrially available, pharmaceutically and technologically easy to process and nontoxic.

In the case of other active agents such as plant protectives, fertilisers or growth regulators, the aforedescribed advantages are to be realised in accordance with the individual active agent.

These objects are achieved by the invention.

Thus the subject matter of the invention is a system for the controlled release of active agents, composed of a core containing at least one soluble active agent and adjuvants and of a shell composed of a film former and a soluble, hydrophilic or hydrophobic plasticiser, which is characterised in that the core contains at least one osmotically active adjuvant and at least one polymeric adjuvant capable of swelling, which adjuvant has a less pronounced tendency to hydratise than the active agents and osmotically active adjuvants in use and will precipitate in a saturated aqueous solution of the osmotically active adjuvant and/or active agent, the shell being composed of a permeable or semi-permeable wall forming material.

A further subject matter of the invention is a process for the preparation of a system for the controlled release of active agents which is characterised in that at least one soluble active agent is mixed with at least one osmotically active substance and at least one polymeric adjuvant capable of swelling and, optionally, with further conventional adjuvants, that the mixture is compressed into cores which are then covered with a shell made of a film former and a soluble, hydrophilic or a hydrophobic plasticiser.

The annexed figures show the following:
- Figure 1: The release of diltiazem from pharmaceutical preparations according to Example 2 ("Release K");
- Figure 2: the plasma level following the administration of the diltiazem pharmaceutical preparation according to Example 2 ("Treatment K") in comparison to a commercial delayed release drug ("Treatment U"); and
- Figure 3: the plasma level of the primary metabolite, desacetylic diltiazem, following the administration of the diltiazem pharmaceutical preparation according to Example 2 ("Treatment K") in comparison to a commercial delayed release drug ("Treatment U").

As regards solubility, a high solubility substance is one to which less than 30 mass parts of solvent have to be added to dissolve 1 mass part thereof; see Arzneibuch der DDR, Akademie Verlag, Berlin 1985, I/II/4.0.

In the system of the invention, the release of the active agents is controlled by compressing it with suitable osmotically active and with osmotically almost inactive substances (a polymeric adjuvant capable of swelling) and then covering the obtained core with suitable wall material, the compatibility of the properties of the wall and core materials being such that in a lag phase, water flows through the membrane which has solute-retaining properties, said water flow being determined by the osmotic activity of the core material.

The system of the invention can exhibit at least one release opening, prepared in the usual manner, for the active agent solution. If no such opening has been prepared, then the hydrostatic pressure that builds up during the lag phase will selectively loosen up the wall structure and induce the formation of micropores (pore size in the »m range). This, in turn, will lead to the active agent solution (which is formed by fluid entering the core as a result of osmosis to dissolve the active agent) being pumped out mainly by means of convective processes. The hydrostatic pressure is the cause of the active agent release.

Examples of suitable adjuvants with enough osmotic activity are lactose, fructose, dextrose, sucrose, mannitol, sodium chloride, potassium chloride, potassium sulphate and mono-, di- and trisodium phosphate and mixtures thereof.

The osmotically active adjuvant can be a substance with buffer activity. If the active agent is osmotically active enough itself, it can take over the function of this adjuvant. In that case, there is no requirement for an additional osmotically active adjuvant.

In the system of the invention, the osmotically active adjuvant is employed in a concentration of 2 to 98 mass percent, based on the total adjuvant mass in the core.

Examples of suitable polymeric adjuvants capable of swelling are polyvinyl alcohols, preferably with a residual acetate content of 6 to 18 mol percent and a mean molar mass of 20,000 to 70,000, and cellulose derivatives such as methyl cellulose, ethyl cellulose, hydroxypropyl cellulose, hydroxypropylmethyl cellulose and carboxymethyl cellulose. These polymeric adjuvants capable of swelling exhibit a less pronounced tendency to hydrate than the employed active agents or combinations of the employed active agents or combinations of the employed active agents with the osmotically active adjuvants. If they are suitable, they will precipitate in a saturated aqueous solution of the osmotically active adjuvant and/or active agent. It is also possible to use other conventional pelleting adjuvants such as suitable binder, fillers and lubricants.

In the system of the invention, the polymeric adjuvant capable of swelling is used in a concentration of 5 to 90, preferably 5 to 60 mass percent, based on the total adjuvant mass in the core.

The shell is composed of suitable film formers known per se such as cellulose acetate, ethyl cellulose, polyvinyl acetate or polyacrylic acid copolymers and soluble, hydrophilic (e.g. polyethylene glycols) or hydrophobic (e.g. rinseed oil) plasticisers. A preferable plasticiser is a polyethylene glycol having a mean molar mass of 400 to 20,000. The plasticiser is employed in an amount of 2 to 30 mass percent, based on the film formers.

The coating is such that it imparts salt-retaining properties, which will be referred to as semipermeable properties, to the shell of the system.

If suitable osmotically active substances with buffer activity are used (e.g. mono- or bi-basic salts of phosphoric acid), a suitable pH environment can be advantageously adjusted in the core of the system in a manner known to those of skill in the art. This is done to also provide optimum solution conditions and, by making use of convective processes, release conditions for pH-dependent soluble active agents, or to influence in the desired manner the pH-dependent swelling of the polymeric adjuvants capable of swelling.

According to the invention, the controlled release of the active agent takes place via at least three different mechanisms:
1. Via the properties of the shell (control membrane) such as thickness, surface, water permeability, pore number and size, all of which can be regulated in a manner known per se,
2. via the properties of the core material whose composition determines the osmotic pressure and the viscosity of the forming active agent/adjuvant solution, and
3. via the ratio of the saturation solubility of the soluble active agents and adjuvants in use.

The polymeric adjuvant capable of swelling which is incorporated into the core material is present under the usual conditions (intact shell) in a "salted-out" condition - i.e. at most slightly hydrated and thus at most slightly dissolved. If the shell is damaged, there is an excess of water that the osmotically active substance can no longer completely bind, and this will cause the adjuvant to swell. The adjuvant thus counteracts the uncontrolled inflow of water and the uninhibited outflow of the rapidly dissolving pharmaceutical agent. This, in turn, causes a decrease in the amount of freely available water, which water is again primarily bound by the osmotically active substance having the higher affinity to water. As a result the adjuvant capable of swelling is at least partially dehydrated and thus returns to its solid form.

For the person of skill in the art it was surprising and unforeseeable that the incorporation of hydrophilic, polymeric adjuvants capable of swelling into the core material of the system according to the invention did not lead to a swelling of the polymer as it is described in EP-A-0 277 092 where it is used to control the release of low solubility pharmaceutical agents, and that it did not lead to a marked increase in the viscosity of the active agent/adjuvant solution in the compartment produced by the semipermeable shell prepared according to conventional and known processes. The person of skill in the art would have expected this to become apparent in an essential decrease in the release of the active agent/adjuvant solution through the pores of the semipermeable shell. It was rather the case that in addition to the advantageous effect of the improved pellability of the core material caused by the use of the hydrophilic, polymeric adjuvants which are capable of swelling and have good binding properties, if the shell is intact, the influence on the active agent release from the system is negligible.
The system of the invention also includes the coating of the core with a membran which is permeable for the dissolved active agent and/or for the dissolved osmotically active adjuvant. It has surprisingly been found that the core composition according to the invention, if coated with a permeable membran, also garantuees the functionality and consequently the safety of the system.
However, in the case of a damaged shell, which will nullify the function, and thus safety, of today's known, conventional membrane controlled/membrane porosity controlled release systems for pharmaceutical agents, it was surprisingly found that the composition according to the invention guaranteed the functionality, and thus safety, of the system. For the person of skill in the art, this has clearly detectable advantages as regards the therapeutic safety and the technologically easy and inexpensive preparation of the system according to the invention, too. The person of skill in the art can expect the release pattern of the active agent or agents to vary if he varies the layer thickness of the shell when carrying out a conventional procedure. It is also common knowledge that the layer must have a minimum thickness in order to obtain sufficiently long retardation and, in particular, a linearization of the release curve (referred to a release according to zero-order kinetics in this description). It is typical for a lag phase to occur during release which lasts for a more or less long period of time and in which no active agents, or only therapeutically insignificant amounts thereof, are released (K. Heilmann, Therapeutische Systeme, Georg Thieme, Stuttgart 1978).
The procedure according to the invention and the composition of the pharmaceutical preparation can achieve the advantageous linearization of the release pattern even in the case of minimal layer thicknesses.

The system of the invention can be present in the form of conventional formulations such as tablets or capsules (single unit drug dosage forms). It can also be a multicompartment form, or a part thereof, and, for example, be filled into a capsule. The multicompartment form means dividing the total dose into several small units (microforms such as microcapsules, pellets and microtablets; small microunits, usually having a size of under 3 mm, obtained by various preparation processes, e.g. coazervation, extrusion, compression, tabletting).

Specific examples of the high solubility active agents that are suitable for the release system are pholedrine sulphate and diltiazem. According to the teaching of this application, each of these active agents enables the preparation of advantageous controlled release systems if the adjuvants are used in the defined ratios.

In the following, the invention is to be explained for the example of the active agent pholedrine sulphate (which is used for a soluble pharmaceutical agent), for the osmotically active compound potassium chloride (as an example of the numerous possible osmotically active compounds) and the soluble polymer polyvinyl alcohol (PVA) capable of swelling, type 55/12, VEB Chem. Werke Buna, Schkopau, German Democratic Republic (as an example of a polymeric adjuvant capable of swelling and having a less pronounced tendency to hydrate than the osmotically active component(s)) as the core components, cellulose-2,5-acetate as the film former and polyethylene glycol PEG 600 as the hydrophilic plasticiser:

### Example 1:

### Shell composition:

| | |
|---|---|
| Cellulose-2,5-acetate | 100 mass parts |
| Polyethylene Glycol 600 | 25 mass parts |

are placed in an acetonic solution and applied to the core up to a shell thickness of 60 »m using conventional methods.

### Core Prescription 1a

| | |
|---|---|
| Pholedrine Sulfate | 60.0 mg |
| Potassium Chloride | 190.0 mg |
| Total Mass | 250.0 mg |

are compressed to formed bodies having a diameter of 7 mm using conventional procedures.

### Core Prescription 1b

| | |
|---|---|
| Pholedrine Sulfate | 60.0 mg |
| Potassium Chloride | 165.0 mg |
| PVA 55/12 | 25.0 mg |
| Total Mass | 250.0 mg |

are compressed to formed bodies having a diameter of 7 mm using conventional procedures.

### Core Prescription 1c

| | |
|---|---|
| Pholedrine Sulphate | 60.0 mg |
| Potassium Chloride | 152.5 mg |
| PVA 55/12 | 36.5 mg |
| Total Mass | 250.0 mg |

are compressed to formed bodies having a diameter of 7 mm using conventional procedures.

### Core Prescription 1d

| | |
|---|---|
| Pholedrine Sulphate | 60.0 mg |
| Potassium Chloride | 127.5 mg |
| PVA 55/12 | 62.5 mg |
| Total Mass | 250.0 mg |

are compressed to formed bodies having a diameter of 7 mm using conventional procedures.

The release pattern of pharmaceutical preparations according to the prescriptions of Example 1 as the released amount of pholedrine sulphate (wt) ± standard deviation (s) in n = 6 determinations (according to USP XXI, Paddle Method, release medium = water):

### 1a

| | | | | | | | | | | | |
|---|---|---|---|---|---|---|---|---|---|---|---|
| t[min] | 0 | 15 | 30 | 60 | 120 | 180 | 240 | 300 | 360 | 420 | 480 |
| wt[mg] | 0 | 0.3 | 0.8 | 3.7 | 30.9 | 45.5 | 52.4 | 55.3 | 56.9 | 57.5 | 58.3 |
| ± s | 0 | 0.2 | 0.5 | 2.3 | 2.8 | 2.0 | 2.3 | 1.0 | 0.5 | 0.3 | 0.1 |

### 1b

| | | | | | | | | | | | |
|---|---|---|---|---|---|---|---|---|---|---|---|
| t[min] | 0 | 15 | 30 | 60 | 120 | 180 | 240 | 300 | 360 | 420 | 480 |
| wt[mg] | 0 | 0.5 | 0.9 | 5.3 | 18.2 | 29.2 | 38.6 | 43.9 | 47.4 | 50.1 | 52.0 |
| ± s | 0 | 0 | 0.1 | 1.5 | 3.8 | 1.4 | 0.8 | 0.9 | 1.1 | 0.4 | 0.5 |

### 1c

| | | | | | | | | | | | |
|---|---|---|---|---|---|---|---|---|---|---|---|
| t[min] | 0 | 15 | 30 | 60 | 120 | 180 | 240 | 300 | 360 | 420 | 480 |
| wt[mg] | 0 | 1.8 | 3.9 | 8.8 | 17.7 | 25.4 | 32.5 | 37.6 | 41.7 | 45.5 | 48.8 |
| ± s | 0 | 0.9 | 0.7 | 0.7 | 0.5 | 1.0 | 2.2 | 1.1 | 0.8 | 0.9 | 0.5 |

### 1d

| | | | | | | | | | | | |
|---|---|---|---|---|---|---|---|---|---|---|---|
| t[min] | 0 | 15 | 30 | 60 | 120 | 180 | 240 | 300 | 360 | 420 | 480 |
| wt[mg] | 0 | 0.8 | 1.8 | 3.6 | 10.0 | 15.8 | 20.8 | 26.6 | 33.7 | 38.8 | 43.2 |
| ± s | 0 | 0.2 | 1.4 | 1.0 | 1.0 | 0.7 | 1.1 | 2.9 | 0.7 | 1.8 | 0.4 |

At first, there is no pholedrine sulphate release, at most for a very short period of time, according to the desired zero-order kinetics at this layer thickness (60»m). This is due to the fact that the minimum layer thickness necessary for the functionality of an osmotically releasing system has not yet been achieved.

However, the higher the amount of PVA, the greater the linearization of the curves. Prescription 1d contains 25% of polyvinyl alcohol and thus comes very close to the ideal release profile of zero-order kinetics.

It is, however, surprising for the person of skill in the art that the large amount of cold water-soluble polyvinyl alcohol capable of swelling does not cause the thin shell to burst or initiate an extreme decrease in the release rate of the incorporated pharmaceutical agent.

The following will describe the release pattern of prescription 1d after the only 60 »m thick shell had been damaged. Additional openings, having a diameter of up to 3000 »m, prepared in the shell were to have a strong effect on or nullify the functionality of the pharmaceutical agent release system, which is only 7 mm in diameter itself, in a manner to be expected by those of skill in the art. This can be proved in control tests using known osmotically releasing systems or pharmaceutical preparations that were not prepared according to the process of the invention.

The release pattern of pharmaceutical preparations according to prescription 1d of Example 1 as the released amount of pholedrin sulphate (wt) ± standard deviation in n = 6:
With no release opening:

### 1d

| | | | | | | | | | | |
|---|---|---|---|---|---|---|---|---|---|---|
| t[min] | 0 | 15 | 30 60 | 120 | 180 | 240 | 300 | 360 | 420 | 480 |
| wt[mg] | 0 | 0.8 | 1.8 3.6 | 10.0 | 15.8 | 20.8 | 26.6 | 33.7 | 38.8 | 43.2 |
| ± s | 0 | 0.2 | 1.4 1.0 | 1.0 | 0.7 | 1.1 | 2.9 | 0.7 | 1.8 | 0.4 |

With membrane damage 640 »m:

| | | | | | | | | | | |
|---|---|---|---|---|---|---|---|---|---|---|
| t[min] | 0 | 15 | 30 | 60 120 | 180 | 240 | 300 | 360 | 420 | 480 |
| wt[mg] | 0 | 1.8 | 5.5 | 9.3 16.3 | 22.6 | 29.6 | 35.4 | 41.5 | 45.7 | 48.2 |
| ± s | 0 | 1.5 | 2.6 | 1.3 2.0 | 1.1 | 1.9 | 1.5 | 0.4 | 0.7 | 0.2 |

With membrane damage 3000 »m:

| | | | | | | | | | | | |
|---|---|---|---|---|---|---|---|---|---|---|---|
| t[min] | 0 | 15 | 30 | 60 | 120 | 180 | 240 | 300 | 360 | 420 | 480 |
| wt[mg] | 0 | 2.2 | 7.8 | 13.9 | 22.3 | 29.1 | 36.1 | 41.6 | 46.2 | 49.5 | 51.3 |
| ± s | 0 | 1.3 | 2.3 | 1.1 | 1.4 | 0.9 | 0.3 | 1.3 | 0.8 | 0.9 | 0.4 |

These data show that within 8 hours, the organism given a pharmaceutical agent release system according to the invention received only 8 mg of the total dosis of 60 mg sooner. The following figures are to give even clearer evidence of these circumstances:

The initial and mean release speed from pharmaceutical preparations according to Example 1, prescription 1d, membrane thickness 60 »m, n = 6

| Membrane Damage [»m] | Initial Release Rate [mg/h] | Mean Release Rate [mg/h] |
|---|---|---|
| Without | 4.2 ± 4.9 | 6.1 ± 0.2 |
| 230 | 7.6 ± 4.5 | 6.0 ± 0.3 |
| 640 | 14.6 ± 9.1 | 6.5 ± 0.7 |
| 1670 | 19.9 ± 7.1 | 6.8 ± 0.5 |
| 3000 | 22.4 ± 6.7 | 7.0 ± 0.5 |

In the system according to the invention, even larger-scale damage in the shell will be compensated for, and this enhances the safety of the pharmaceutical agent administration.

It is of further advantage that the active transport of the incorporated active agent(s) out of the pharmaceutical preparation makes the active agent release more complete, and thus the availability of the active agent is higher than in matrix pharmaceutical preparations, for instance.

The effectivity of the process according to the invention is underlined by the in vitro and in vivo behaviour of the pharmaceutical agent diltiazem illustrated in Example 2.

This example will also serve to prove the in vivo functionality of the system of the invention.

### Example 2

Tablets of the following structure have been prepared:

### Shell Composition

| | |
|---|---|
| Cellulose-2,5-acetate | 225 parts by mass |
| Polyethylene Glycol 600 | 112.5 parts by mass |

are placed in an acetonic solution and applied to the core up to a shell thickness of 30 »m using conventional methods.

### Core Prescription

| | |
|---|---|
| Diltiazem Hydrochloride | 180.0 mg |
| PVA 55/12 | 78.8 mg |
| Potassium Sulphate | 250.2 mg |
| Magnesium Stearate | 1.0 mg |
| Total Mass | 510.00 mg |

are compressed according to conventional procedures into formed bodies having a diameter of 9 mm .

In an open, randomised, double cross-over study, the pharmacokinetic parameters which occurred after the administration of one diltiazem delayed release formulation containing 90 mg and one containing 180 mg of active agent were examined:
- Reference Drug/"Treatment U": 90 mg of a delayed release tablet (commercial drug) Amount of active agent 90 mg of diltiazem
- Test Drug/"Treatment K": Diltiazem retard 180 mg, tablets Prescription according to Example 2, amount of active agent 180 mg of diltiazem
The quantitative determination of diltiazem and its primary metabolite desacetylic diltiazem was carried out in the plasma using a specific, validated HPLC method. The lowest quantification level was 2 ng diltiazem/ml of plasma and 1 ng of desacetylic diltiazem/ml of plasma.
The study included four male volunteers aged between 18 and 40, all of whom completed the study according to instructions.
The test persons appeared on the different occasions as determined by the randomization plan, separated by wash-out periods of one week, to receive administrations of diltiazem. The test persons were bound to a reduced food intake for ten hours before up to four hours after each drug administration.
Immediately prior to drug administration (predose) and after 1, 2, 3, 4, 6, 8, 10, 12, 24, 30, 36 and 48 hours, 10 ml venous blood samples were taken and plasma obtained to determine the concentration of diltiazem and desacetylic diltiazem.

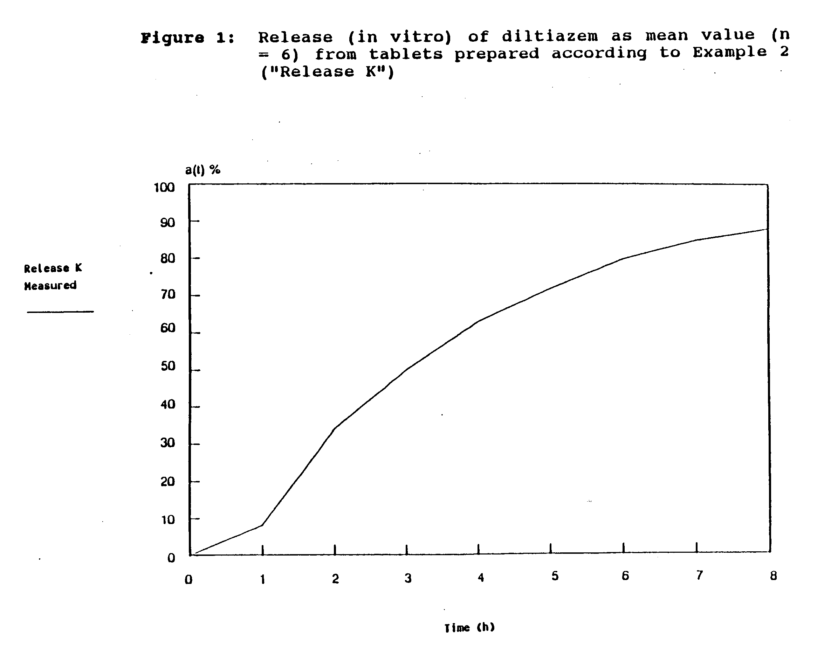

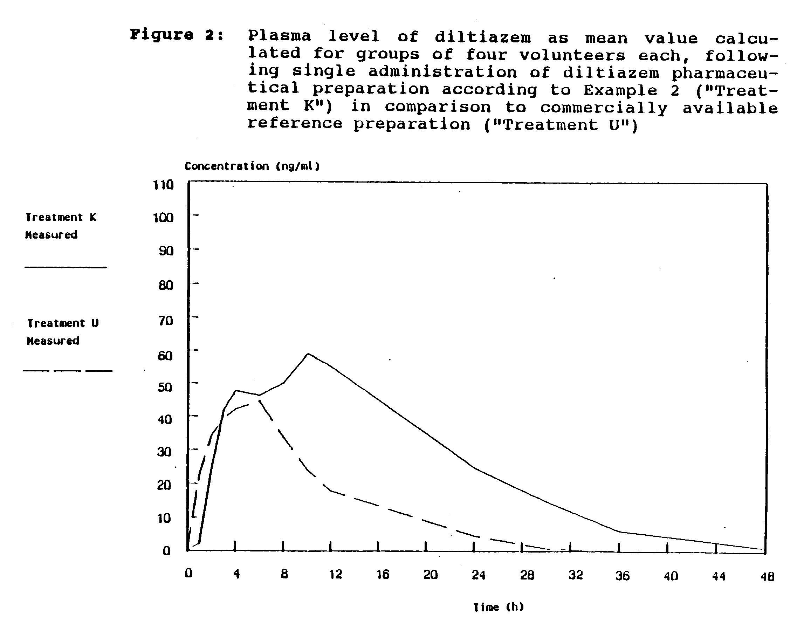

Figure 1 illustrates the in vitro release pattern of diltiazem as the mean value of six determinations of tablets prepared according to Example 2. A lag phase in which active agent release is lower can be seen.
The plasma levels of diltiazem following the administration of the two preparations shown in Figure 2 exhibit the clear advantage of the system according to the invention in view of both the availability and the duration of the minimal therapeutic concentrations, despite the higher dosage according to Example 2. The pharmaceutical preparation according to Example 2 enables single daily doses.

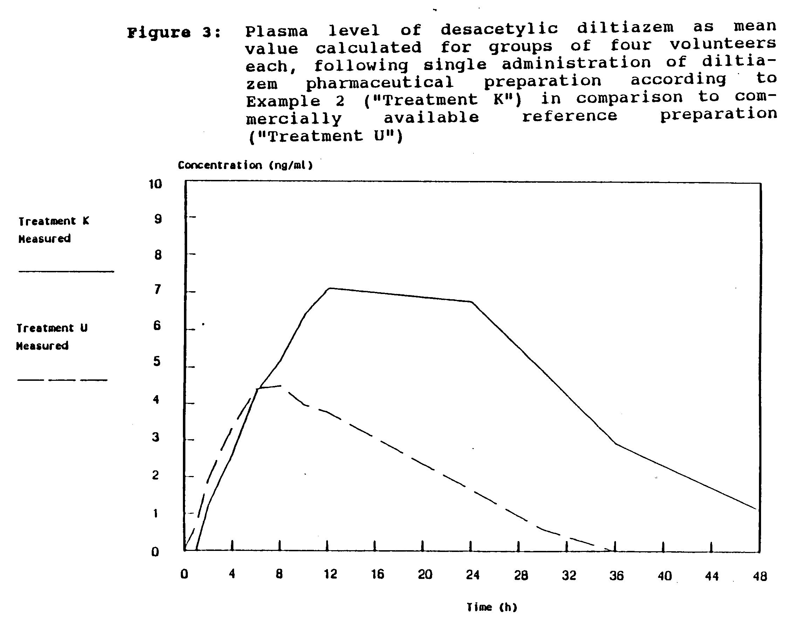

The plasma levels of desacetylic diltiazem shown in Figure 3 prove the plausibility of the analysis method and underline the significance of the results.

In principle, the aforedescribed systems can be used for various active agents. In addition to the active agents used in agricultural chemistry such as fertilisers, plant protectives, e.g. insecticides and growth regulators, these systems can be used with preference for pharmaceutical agents in the field of human and veterinary medicine. The possibility to use these active agents in such a system is limited by their saturation solubility and depot dosage. In this regard, it is the ratio of the saturation solubilities of the osmotically active compound and the active agent or active agent mixture which is of significance. This is because it determines the required amount of adjuvants and thus the total mass of the system and its applicability. If the active agents have very high solubility, it is also possible to incorporate very large doses thereof.
If its solubility is poor, this form of active agent can only be incorporated in relatively small doses.
The use of lower solubility carriers (e.g. potassium sulphate) improves the usability of low solubility active agents.

## Claims

1. A system for the controlled release of active agents, composed of a core containing at least one soluble active agent and adjuvants and of a shell composed of a film former and a soluble, hydrophilic or hydrophobic plasticiser, characterised in that the core contains at least one osmotically active adjuvant and at least one polymeric adjuvant capable of swelling, which adjuvant has a less pronounced tendency to hydratise than the active agents and osmotically active adjuvants in use and will precipitate in a saturated aqueous solution of the osmotically active adjuvant and/or active agent, the shell being composed of a permeable or semi-permeable wall forming material.

2. The system according to claim 1, characterised in that the active agent is a pharmaceutical agent for use in human or veterinary medicine.

3. The system according to claim 1, characterised in that the active agent is a plant protective, a fertiliser or a growth regulator.

4. The system according to any of claims 1 to 3, characterised in that the semipermeable shell exhibits at least one release opening for the active agent.

5. The system according to any of claims 1 to 4, characterised in that the semipermeable shell exhibits a pore structure which is produced by the buildup of hydrostatic pressure in the core.

6. The system according to any of claims 1 to 5, characterised in that the osmotically active adjuvant is a substance with buffer activity.

7. The system according to claim 1 or 6, characterised in that the osmotically active adjuvant comprises lactose, fructose, dextrose, sucrose, mannitol, sodium chloride, potassium chloride, potassium sulphate, mono-, di- and trisodium phosphate or mixtures of the same.

8. The system according to any of claims 1 to 7, characterised in that it contains the osmotically active adjuvant in a concentration of 2 to 98 mass percent, based on the total adjuvant mass.

9. The system according to claims 1 to 8, characterised in that the polymeric adjuvant capable of swelling is a polyvinyl alcohol, preferably with a residual acetate content of 6 to 18% and a mean molar mass of 20,000 to 70,000, and/or a cellulose derivative or a salt thereof.

10. The system according to claim 9, characterised in that the cellulose derivative is methyl cellulose, ethyl cellulose, hydroxypropyl cellulose, hydroxypropylmethyl cellulose or carboxymethyl cellulose,

11. The system according to any of claims 1 to 10, characterised in that it contains the polymeric adjuvant capable of swelling in a concentration of 2 to 90 mass percent, based on the total adjuvant mass.

12. The system according to any of claims 1 to 11, characterised in that the film former for the semipermeable shell is cellulose acetate, ethyl cellulose, polyvinyl acetate or a polyacrylic acid copolymer.

13. The system according to any of claims 1 to 12, characterised in that the plasticiser for the semipermeable shell is a polyethylene glycol which has a mean molar mass of 400 to 20,000 and is used in an amount of 2 to 30 mass percent, based on the film former.

14. The system according to any of claims 1 to 13, characterised in that it is present, at least partially, in the multicompartment form.

15. The system according to any of claims 1 to 13, characterised in that it is present as a microform.

16. A process for the preparation of a system for the controlled release of active agents according to claim 1, characterised in that at least one soluble active agent is mixed with at least one osmotically active substance and at least one polymeric adjuvant capable of swelling, optionally with further conventional adjuvants, in that the mixture is compressed to form cores, which cores are coated with a shell made of a film former and a soluble, hydrophilic or hydrophobic plasticiser.

## Patentansprüche

1. System für die kontrollierte Freisetzung von Wirkstoffen, bestehend aus einem mindestens einen löslichen Wirkstoff sowie Hilfsstoffe enthaltenden Kern und einer Hülle aus einem Filmbildner und einem löslichen, hydrophilen oder hydrophoben Weichmacher, dadurch gekennzeichnet, daß der Kern mindestens einen osmotisch aktiven Hilfsstoff und mindestens einen quellfähigen polymeren Hilfsstoff enthält, der eine geringere Hydratisierungsneigung hat als die eingesetzten Wirkstoffe und osmotisch aktiven Hilfsstoffe und der in einer gesättigten wässrigen Lösung des osmotisch aktiven Hilfsstoffs und/oder des Wirkstoffs ausfällt, wobei die Hülle aus einem permeablen oder semipermeablen wandbildenden Material zusammengesetzt ist.

2. System nach Anspruch 1, dadurch gekennzeichnet, daß es sich bei dem Wirkstoff um einen Arzneistoff für die Human- oder Veterinärmedizin handelt.

3. System nach Anspruch 1, dadurch gekennzeichnet, daß es sich bei dem Wirkstoff um ein Pflanzenschutzmittel, ein Düngemittel oder um einen Wachstumsregulator handelt.

4. System nach einem der Ansprüche 1 bis 3, dadurch gekennzeichnet, daß die semipermeable Hülle mindestens eine Freigabeöffnung für den Wirkstoff aufweist.

5. System nach einem der Ansprüche 1 bis 4, dadurch gekennzeichnet, daß die semipermeable Hülle eine durch den sich im Kern aufbauenden hydrostatischen Druck erzeugte Porenstruktur aufweist.

6. System nach einem der Ansprüche 1 bis 5, dadurch gekennzeichnet, daß der osmotisch aktive Hilfsstoff ein Stoff mit Pufferwirkung ist.

7. System nach Anspruch 1 oder 6, dadurch gekennzeichnet, daß es als osmotisch aktiven Hilfsstoff Lactose, Fructose, Dextrose, Sucrose, Mannit, Natriumchlorid, Kaliumchlorid, Kaliumsulfat, Mono-, Di- und Trinatriumphosphat oder deren Gemische enthält.

8. System nach einem der Ansprüche 1 bis 7, dadurch gekennzeichnet, daß es den osmotisch aktiven Hilfsstoff in einer Konzentration von 2 bis 98 Masseprozent, bezogen auf die Gesamtmasse der Hilfsstoffe, enthält.

9. System nach Anspruch 1 bis 8, dadurch gekennzeichnet, daß der guellfähige polymere Hilfsstoff ein Polyvinylalkohol, bevorzugt mit einem Restacetatgehalt von 6 bis 18% und einer mittleren Molmasse von 20 000 bis 70 000 und/oder ein Cellulosederivat oder ein Salz davon ist.

10. System nach Anspruch 9, dadurch gekennzeichnet, daß das Cellulosederivat Methylcellulose, Ethylcellulose, Hydroxypropylcellulose, Hydroxypropylmethylcellulose oder Carboxymethylcellulose ist.

11. System nach einem der Ansprüche 1 bis 10, dadurch gekennzeichnet, daß es den quellfähigen polymeren Hilfsstoff in einer Konzentration von 2 bis 90 Masseprozent, bezogen auf die Gesamtmasse der Hilfsstoffe, enthält.

12. System nach einem der Ansprüche 1 bis 11, dadurch gekennzeichnet, daß der Filmbildner für die semipermeable Hülle Celluloseacetat, Ethylcellulose, Polyvinylacetat, oder ein Polyacrylsäure-Copolymerisat ist.

13. System nach einem der Ansprüche 1 bis 12, dadurch gekennzeichnet, daß der Weichmacher für die semipermeable Hülle ein Polyethylenglycol mit einer mittleren Molmasse von 400 bis 20 000 ist, das in einer Menge von 2 bis 30 Masseprozent, bezogen auf den Filmbildner, eingesetzt wird.

14. System nach einem der Ansprüche 1 bis 13, dadurch gekennzeichnet, daß es mindestens zu einem Teil als Mehrkompartimentform vorliegt.

15. System nach einem der Ansprüche 1 bis 13, dadurch gekennzeichnet, daß es als Mikroform vorliegt.

16. Verfahren zur Herstellung eines Systems für die kontrollierte Freisetzung von Wirkstoffen gemäß Anspruch 1, dadurch gekennzeichnet, daß man mindestens einen löslichen Wirkstoff mit mindestens einem osmotisch aktiven Stoff und mindestens einem quellfähigen polymeren Hilfstoff sowie gegebenenfalls mit weiteren üblichen Hilfsstoffen vermischt, das Gemisch zu Kernen preßt und diese mit einer Hülle aus einem Filmbildner und einem löslichen, hydrophilen oder hydrophoben Weichmacher überzieht.

## Revendications

1. Système de libération contrôlée d'agents actifs constitué d'un coeur contenant au moins un agent actif soluble et des adjuvants, ainsi que d'une enveloppe constituée d'un agent filmogène et d'un plastifiant soluble hydrophile ou hydrophobe, caractérisé en ce que le coeur contient au moins un adjuvant osmotiquement actif et au moins un adjuvant polymère susceptible d'un gonflement, lequel adjuvant a une tendance moins nette à s'hydrater que les agents actifs et les adjuvants osmotiquement actifs pendant l'utilisation et il précipitera dans une solution aqueuse saturée de l'adjuvant osmotiquement actif et/ou de l'agent actif l'enveloppe étant constituée d'une substance formant une paroi perméable ou semi-perméable.

2. Système selon la revendication 1, caractérisé en ce que l'agent actif est un agent pharmaceutique à utiliser dans la médecine humaine ou vétérinaire.

3. Système selon la revendication 1, caractérisé en ce que l'agent actif est un protecteur de végétaux, un fertilisant ou un régulateur de la croissance.

4. Système selon l'une quelconque des revendications 1 à 3, caractérisé en ce que l'enveloppe semi-perméable présente au moins une ouverture de libération pour l'agent actif.

5. Système selon l'une quelconque des revendications 1 à 4, caractérisé en ce que l'enveloppe semi-perméable présente une structure de pore qui est produite par la remontée de pression hydrostatique dans le coeur.

6. Système selon l'une quelconque des revendications 1 à 5, caractérisé en ce que l'adjuvant osmotiquement actif est une substance présentant une activité tampon.

7. Système selon la revendication 1 ou 6, caractérisé en ce que l'adjuvant osmotiquement actif comprend le lactose, le fructose, le dextrose, le saccharose, le mannitol, le chlorure de sodium, le chlorure de potassium, le sulfate de potassium, le phosphate monosodique, disodique ou trisodique ou des mélanges de ces derniers.

8. Système selon l'une quelconque des revendications 1 à 7, caractérise en ce qu'il contient l'adjuvant osmotiquement actif en une concentration comprise entre 2 et 98 % en masse par rapport à la masse totale des adjuvants.

9. Système selon les revendications 1 à 8, caractérisé en ce que l'adjuvant polymère susceptible d'un gonflement est un poly(alcool vinylique), de préférence avec une teneur résiduelle en acétate comprise entre 6 et 18 % et une masse molaire moyenne comprise entre 20 000 et 70 000 et/ou un dérivé de la cellulose ou un de ses sels.

10. Système selon la revendication 9, caractérise, en ce que le dérive de la cellulose est la méthylcellulose, l'éthylcellulose, l'hydroxypropylcellulose, l'hydroxypropylméthylcellulose ou la carboxyméthylcellulose.

11. Système selon l'une quelconque des revendications 1 à 10, caractérisé en ce qu'il contient l'adjuvant polymère susceptible d'un gonflement en une concentration comprise entre 2 et 90 % en masse par rapport à la masse totale des adjuvants.

12. Système selon l'une quelconque des revendications 1 à 11, caractérisé en ce que l'agent filmogène destiné à l'enveloppe semi-perméable est l'acétate de cellulose, l'éthylcellulose, le poly(acétate de vinyle) ou un copolymère d'acide polyacrylique.

13. Système selon l'une quelconque des revendications 1 à 12, caractérisé eu ce que le plastifiant destiné à l'enveloppe semi-perméable est un polyéthylèneglycol qui possède une masse molaire moyenne comprise entre 400 et 20 000 et qu'on l'utilise à raison de 2 à 30 % en masse par rapport à l'agent filmogène.

14. Système selon l'une quelconque des revendications 1 à 13, caractérisé en ce qu'il est présent, au moins partiellement, sous la forme à plusieurs compartiments.

15. Système selon l'une quelconque des revendications 1 à 13, caractérisé en ce qu'il est présent en tant que microforme.

16. Procédé de préparation d'un système de libération contrôlée d'agents actifs selon la revendication 1, caractérisé en ce qu'on mélange au moins un agent actif soluble avec au moins une substance osmotiquement active et au moins un adjuvant polymère susceptible d'un gonflement, éventuellement avec d'autres adjuvants classiques, en ce qu'on compresse le mélange pour former des coeurs, lesquels coeurs sont revêtus d'une enveloppe constituée d'un agent filmogène et d'un plastifiant soluble hydrophile ou hydrophobe.
